(19) Europäisches Patentamt
European Patent Office
Office européen des brevets

(11) Veröffentlichungsnummer: **0 011 092**
**B1**

(12) **EUROPÄISCHE PATENTSCHRIFT**

(45) Veröffentlichungstag der Patentschrift: **23.06.82**

(21) Anmeldenummer: **79103007.5**

(22) Anmeldetag: **17.08.79**

(51) Int. Cl.³: **C 07 D 295/14,**
C 07 D 249/06,
C 07 D 233/36,
C 07 D 233/38,
C 07 C 101/447,
A 61 K 31/195,
A 61 K 31/40,
A 61 K 31/41,
A 61 K 31/445,
A 61 K 31/415,
A 61 K 31/50

# (54) Alpha-Aminophenylessigsäurederivate zur Verwendung bei der Behandlung von Entzündungen sowie neue alpha-Aminophenylessigsäurederivate.

(30) Priorität: **22.08.78 DE 2836613**

(43) Veröffentlichungstag der Anmeldung:
**28.05.80 Patentblatt 80/11**

(45) Bekanntmachung des Hinweises auf die Patenterteilung:
**23.06.82 Patentblatt 82/25**

(84) Benannte Vertragsstaaten:
**AT BE CH DE FR GB IT NL SE**

(56) Entgegenhaltungen:
**DE - A - 1 795 151**

**BIOCHEMISTRY, Vol. 5, Nr. 1, Januar 1966, Easton, A. H. NEIMS: "Studies on crystalline D-AMINOACID OXIDASE III Substrate specificity and T-e relationship", Seiten 203—213**

**JOURNAL OF THE CHEMICAL SOCIETY 1955, London, W. DAVIS et al.: "Aryl-2-Halogeno-alkylamines. Part XIII. Chloro-ethylamino-derivatives of some phenoxyalkanoic acids and of some substituted alpha-amino-acids"**

(73) Patentinhaber: **BAYER AG**
**Zentralbereich Patente, Marken und Lizenzen**
**D-5090 Leverkusen 1, Bayerwerk (DE)**

(72) Erfinder: **Schmidt, Gunter, Dr.**
**Pahlkestrasse 63**
**D-5600 Wuppertal 1 (DE)**
Erfinder: **Opitz, Wolfgang, Dr.**
**Silesiusstrasse 80**
**D-5000 Köln 80 (DE)**
Erfinder: **Jacobi, Haireddin, Dr.**
**Finkenweg 2**
**D-5653 Leichlingen 1 (DE)**

(56) Entgegenhaltungen:
Die Akte enthält technische Angaben die nach dem Eingang der Anmeldung eingereicht wurden und die nicht in dieser Patentschrift enthalten sind.

**EP 0 011 092 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

$\alpha$-Aminophenylessigsäure derivate zur Verwendung bei der Behandlung von Entzündungen sowie neue $\alpha$-Aminophenylessigsäurederivate.

Die vorliegende Erfindung betrifft $\alpha$-Aminophenylessigsäurederivate zur Verwendung bei der Behandlung von Entzündungen, sowie einige neue $\alpha$-Aminophenylessigsäurederivate, nämlich $\alpha$ - Amino - $\alpha$ - (4 - thiomorpholino - phenyl)essigsäure, DL - $\alpha$ - [(3 - Methylsulfonyl - imidazolidin - 1 - on - 2 - yl)-carbonylamino] - $\alpha$ - [4 - (3 - methylsulfonyl - imidazolidin - 1 - on - 2 - yl) - carbonylamino - phenyl]-essigsäure und D - $\alpha$ - Amino - $\alpha$ - [4 - imidazolidin - 2 - on - 1 - yl)phenyl]essigsäure. Es ist bereits bekannt, daß Phenylessigsäurederivate pharmakologische Wirkungen besitzen. In den US-Patenten 3 692 819, 3 767 800, 3 766 260, 3 868 391, 3 936 467, 3 997 669; in dem britischen Patent 1 299 172 und in dem niederländischen Patent NE 68, 10359 werden Phenylessigsäurederivate beschrieben, die unter anderem entzündungshemmende und antipyretische Eigenschaften besitzen. Entsprechende Aminosäurederivate sind bisher nicht bekannt geworden.

Die erfindungsgemäßen $\alpha$-Aminophenylessigsäurederivate zur Verwendung bei der Behandlung von Entzündungen werden durch die allgemeine Formel I beschrieben,

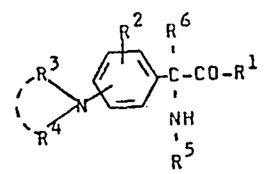

(I)

in welcher

$R^1$ für Hydroxy oder Alkoxy mit 1 bis 4 Kohlenstoffatomen steht,

$R^2$ für Wasserstoff, Alkyl mit 1 bis 4 Kohlenstoffatomen, Trifluormethyl oder Halogen steht,

$R^3$ und $R^4$ gleich oder verschieden und jeweils für Wasserstoff, Allyl oder Alkyl mit bis zu 4 Kohlenstoffatomen stehen, wobei die Alkylreste gegebenenfalls substituiert sind durch Amino, Cyano oder Alkoxy mit 1 bis 2 Kohlenstoffatomen

oder

$R^3$ und $R^4$ gemeinsam mit dem Stickstoffatom einen 5- bis 7-gliedrigen aliphatischen Ring bilden, der gegebenenfalls gesättigt oder ungesättigt sein kann, und der ein Sauerstoffatom, ein Schwefelatom, eine SO-Gruppierung, eine $SO_2$-Gruppierung oder eine N—R'''-Gruppierung, wobei R''' für Wasserstoff, Alkyl mit 1 bis 2 Kohlenstoffatomen oder Methoxyäthyl steht, enthalten kann oder gemeinsam mit dem Stickstoffatom einen Triazolylrest bilden,

$R^5$ für Wasserstoff oder Alkyl mit 1 bis 4 Kohlenstoffatomen steht und

$R^6$ für Wasserstoff oder Alkyl mit 1 bis 2 Kohlenstoffatomen steht.

Die Erfindung betrifft auch die pharmakologisch unbedenklichen Salze dieser Verbindungen zur Verwendung bei der Behandlung von Entzündungen, Arzneimittel, die mindestens eine Verbindung der Formel I oder eines ihrer pharmakologisch unbedenklichen Salze enthalten, ein Verfahren zur Herstellung dieser Arzneimittel sowie ein Verfahren zur Herstellung von $\alpha$-Amino-$\alpha$-(4-thiomorpholino-phenyl)-essigsäure.

Die erfindungsgemäßen Verbindungen können bezüglich des Chiralitätszentrums C* in den beiden möglichen R(D)- and S(L)-Konfigurationen und als racemische Gemische (DL) vorliegen.

Es ist bekannt, daß Substanzen aus der Indolreihe starke antiphlogistische und antipyretische Eigenschaften besitzen. Diese bekannten Antiphlogistika besitzen jedoch den Nachteil, daß sie entweder unerwünschte Nebenwirkungen zeigen oder nur eine geringe therapeutische Aktivität besitzen und dann in großen Mengen verabreicht werden müssen. Die erfindungsgemäßen Verbindungen zeigen neben einer starken therapeutischen Aktivität nur sehr geringe Nebenwirkungen und stellen somit eine Bereicherung der Pharmazie dar.

Man erhält die $\alpha$-Aminophenylessigsäurederivate der Formel I, wenn man substituierte Benzaldehydderivate der Formel II

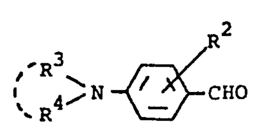

(II)

in welcher

$R^2$, $R^3$ und $R^4$ die oben angegebene Bedeutung haben,

mit Natriumcyanid und Ammoniumcarbonat zu Hydantoinderivaten der allgemeinen Formel III

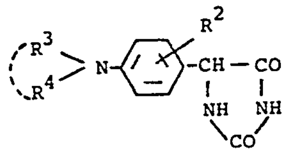

(III)

umsetzt und diese dann zu den entsprechenden Aminosäuren der allgemeinen Formel I hydrolysiert und anschließend diese Aminosäuren gegebenenfalls in an sich bekannter Weise an der Carboxylgruppe verestert oder amidiert und/oder an der Aminogruppe acyliert. Die Umsetzung der Hydantoinverbindungen der allgemeinen Formel III zu den $\alpha$-Aminophenylessigsäureverbindungen der Formel I erfolgt in an sich bekannter Weise durch saure oder alkalische Hydrolyse.

Die erfindungsgemäßen Verbindung existieren in stereoisomeren Formen. Sowohl die Antipoden als auch die Racematformen und die Diastereomerengemische sind Gegenstand der vorliegenden Erfindung. Die Racemate lassen sich ebenso wie die Diastereomeren in bekannter Weise in die stereoisomereinheitlichen Bestandteile trennen (vgl. E. L. Eliel, Stereochemistry of Carbon Compounds, McGraw Hill, 1962).

Je nach Art der verwendeten Ausgangsstoffe kann die Synthese der erfindungsgemäßen Verbindungen beispielhaft durch folgendes Formelschema wiedergegeben werden:

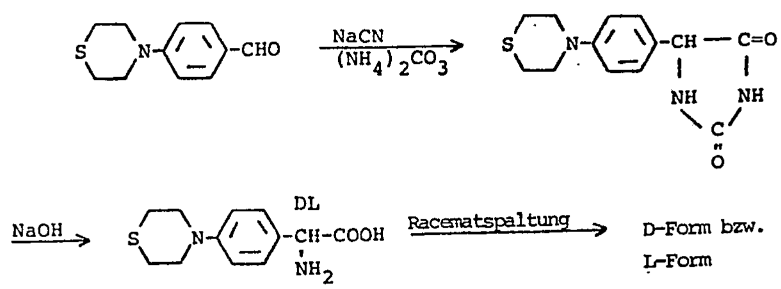


Die als Ausgangsstoffe einsetzbaren Aldehyde der allgemeinen Formel II sind bekannt oder können nach bekannten Methoden hergestellt werden, indem man z.B. entsprechende substituierte Anilinderivate nach Vilsmeier mit Phosphoroxichlorid und Dimethylformamid zu Aldehyden umsetzt (vgl. G. A. Olah et al., Friedel-Crafts and Related Reactions, Vol. III, Part 2, 1153—1256). Außerdem können die Verbindungen der Formel II erhalten werden, indem man entsprechende Aminobenzaldehyde in an sich bekannter Weise an der Aminfunktion variiert.

Als Verdünnungsmittel kommen bei dem erfindungsgemäßen Verfahren alle inerten organischen Lösungsmittel, Wasser und Gemische hiervon in Frage. Hierzu gehören vorzugsweise Alkohole wie Methanol, Äthanol, Isopropanol, Äther wie Dioxan, Diäthyläther, Tetrahydrofuran, Glykolmonomethyläther oder Dimethylformamid, Dimethylsulfoxid und Acetonitril.

Die Reaktionstemperaturen können in einem größeren Bereich variiert werden. Im allgemeinen arbeitet man zwischen 10 und 120°C, vorzugsweise im Bereich zwischen 25 und 100°C.

Bei der Durchführung des erfindungsgemäßen Verfahrens wird Natriumcyanid und Ammoniumcarbonat vorzugsweise im Überschuß mit dem Aldehyd der Formel II umgesetzt. Besonders vorteilhaft ist es, diese Reaktanten in etwa 2-bis 5-fachem Überschuß einzusetzen.

Die erfindungsgemäßen Wirkstoffe können in üblicher Weise in die üblichen Formulierungen überführt werden, wie Tabletten, Kapseln, Dragees, Pillen, Granulate, Aerosole, Sirups, Emulsionen, Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,1 bis 95 Gew.-%, insbesondere von 0,5 bis 90 Gew.-%, der Gesamtmischung vorhanden sein, d.h. in Mengen die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt: Wasser, nichttoxische organische Lösungsmittel, wie Paraffine (z.B. Erdölfraktionen), pflanzliche Öle (z.B. Erdnuß-/Sesamöl), Alkohole (z.B. Äthylalkohol, Glycerin), Glykole (z.B. Propylenglykol, Polyäthylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nichtionogene anionische Emulgatoren (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaugen, Methylcellulose, Stärke und

Polyvinylpyrrolidon) und Gleitmittel (z.B. Magnesiumstearat, Talkum, Stearinsäure und Natriumlaurylsulfat).

Die Applikation erfolgt in üblicher Weise oral oder parenteral, insbesondere perlingual, intravenös oder intramuskulär. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen wie Stärke, vorzugsweise Kartoffelstärke, Gelatine und dergleichen enthalten.

Weiterhin können Gleitmittel wie Magnesiumstearat, Natriumlaurylsulfat und Talkum zum Tablettieren mitverwendet werden. Im Falle wäßriger Suspension und/oder Elixieren, die für orale Anwendung gedacht sind, können die Wirkstoffe außer mit den obengenannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 5 mg/kg Körpgergewicht pro Tag zur Erzielung wirksamer Ergebnisse zu verabreichen, und bei oraler Applikation beträgt die Dosierung etwa 0,01 bis 20 mg/kg, vorzugsweise 0,1 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

### Beispiel 1

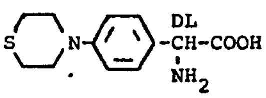

a) DL - $\alpha$ - Amino - $\alpha$ - [4 - (tetrahydro - 1,4 - thiazinyl)phenyl]essigsäure oder DL - $\alpha$ - Amino- $\alpha$ - (4 - thiomorpholino - phenyl)essigsäure

105 g (0,378 Mol) 5-[4-(tetrahydro-1,4-thiazinyl)phenyl]-2,4-imidazolidindion werden in 720 ml 10%iger Natronlauge unter Rühren 20 Stunden auf 100°C erhitzt. Man kühlt auf 0°C ab, säuert mit konzentrierten Salzsäure auf pH 1 und rührt noch 10 Minuten nach. Anschließend wird die Lösung mit 4 N NaOH bei Eiskühlung auf einen pH-Wert von 5,0 eingestellt. Das hellgelbe ausgefallene Material wird abgesaugt und gründlich mit Wasser gewaschen. Nach dem Trocknen bei 50°C erhält man 90 g Rohprodukt, das zweimal in siedendem Äthanol verrieben, danach abgesaugt und mit Äther gewaschen wird.

Ausbeute: 84,5 g (83%).

$C_{12}H_{16}N_2O_2S \cdot H_2O$ (270,3)

| | | | | |
|---|---|---|---|---|
| Ber. | C 53,32 | H 6,71 | N 10,37 | S 11,88 |
| Gef. | C 54,4 | H 6,2 | N, 10,7 | S 12,1 |

$^1$H-NMR (DCOOD, 60 MHz): $\delta$ = 3,10—3,25 (m; 3-$CH_2$, 5-$CH_2$, Thiazin), 3,90—4,06 (m; 2-$CH_2$, 6-$CH_2$, Thiazin), 5,42 (s; $\alpha$-CH), 7,74 ppm (s; 4H, Phenyl).

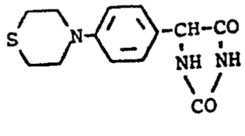

b) 5-[4-(Tetrahydro-1,4-thiazinyl)phenyl]-2,4-imidazolidindion

102 g (0,47 Mol) 4-(Thiomorpholino)benzaldehyd, gelöst in 1200 ml Äthanol, werden zu einer Suspension von 34,8 g (0,71 Mol) Natriumcyanid und 184 g (1,91 Mol) Ammoniumcarbonat in 1200 ml Wasser in einem 4 l Dreihalskolben bei 60°C getropft und 30 Stunden bei 60°C gerührt. Aus der

anfänglich klaren hellgrünen Lösung fällt über Nacht ein helles Material aus. Nach Abdestillieren des Äthanols im Vakuum wird die Restlösung mit konzentrierter Salzsäure auf pH 2 angesäuert. Das suspendierte Material geht dabei in Lösung. Die klare Lösung wird 15 Minuten gerührt und bei 5°C mit 4 N NaOH auf pH 7 eingestellt. Das ausgefallene Material wird abgesaugt und mit Wasser gewaschen. Das trocken gesaugte Produkt wird dann dreimal mit Äther verrieben, abgesaugt und bei 60°C getrocknet.

Rohausbeute: 185 g.

Das Rohprodukt wird in 700 ml heißem Äthanol verrieben, heiß abgesaugt und mit Äthanol gewaschen.

Ausbeute: 105 g (81%).

$C_{13}H_{15}N_3O_2S$ (277,3)

| | | | | |
|---|---|---|---|---|
| Ber. | C 56,31 | H 5,45 | N 15,15 | S 11,56 |
| Gef. | C 56,4 | H 5,8 | N 15,5 | S 11,4 |

$^1$H-NMR ([$D_6$]DMSO, 60 MHz): $\delta = 2,63—2,72$ (d; 3-$CH_2$, 5-$CH_2$, Thiazin), 3,49—3,60 (d; 2-$CH_2$, 6-$CH_2$, Thiazin), 5.03 (s; 5-H), 6,83—7,27 ppm (AA'BB'-System, 4H).

Beispiel 2

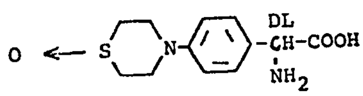

a) DL-$\alpha$-Amino-$\alpha$-[4-(1-oxo-thiomorpholin-4-yl)phenyl]essigsäre

Eine Lösung von 2,7 g (0,01 Mol) DL-$\alpha$-Amino-$\alpha$-(4-thiomorpholinophenyl)esigsäure in 30 ml 1 N Salzsäure wird mit 11,3 ml (0,01 Mol) 3%iger Wasserstoffperoxid-Lösung versetzt und 90 Minuten bei Raumtemperatur aufbewahrt. Nach Abfiltrieren geringer ungelöster Anteile wird die Lösung auf eine Ionenaustauscher-Säule (Amberlite IR—120, $H^+$-Form) gegeben. Nach dem Neutralwaschen der Säule mit Wasser wird das Produkt mit 2 N Ammoniaklösung eluiert. Das Eluat wird stark eingeengt, mit Äthanol versetzt, der ausgefallene Niederschlag abfiltriert, mit Äther gewaschen und getrocknet. Ausbeute 1,4 g. Durch Konzentrieren der Mutterlauge und erneutes Fällen mit Äthanol kann man weitere 0,45 g Sulfoxid gewinnen.

Ausbeute: 1,85 g (70%).

Schmelzpunkt: 204°C.

$C_{12}H_{16}N_2O_3S$ (268,3)

| | | | | |
|---|---|---|---|---|
| Ber. | C 53,71 | H 6,01 | N 10,44 | S 11,95 |
| Gef. | C 52,96 | H 6,17 | N 10,42 | S 11,75 |

Beispiel 3

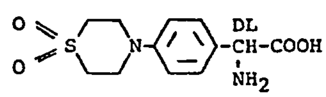

a) DL-$\alpha$-Amino-$\alpha$-(4-thiomorpholinophenyl)essigsäure-S.S-dioxid

162 g (0,524 Mol) 5-(4-Thiomorpholinophenyl)-2,4-imidazolidindion-S.S-dioxid werden in 1300 ml 10%iger Natronlauge 40 Stunden bei 100°C gerührt. Nach Abkühlen auf 0°C wird die Lösung mit konzentrierter Salzsäure auf pH 2 angesäuert, danach auf pH 4,6 zurückgestellt und nach längerem Stehen von einem Niederschlag abgetrennt. Das Filtrat wird auf ungefähr 1 l Volumen eingeengt und 1500 ml Äthanol hinzugefügt. Das ausgefallene Produkt wird abgesaugt und mit Äthanol gewaschen.

Ausbeute: 167 g (112%; enthält 24% Natriumchlorid)

$C_{12}H_{16}N_2O_4S$ (284,3)

| | | | | | |
|---|---|---|---|---|---|
| Ber. | C 38,5 | H 4,3 | N 7,5 | S 8,57 | |
| Gef. | C 37,6 | H 4,3 | H 7,3 | S 7,9 | Cl 14,6 |

$^1$H-NMR ([$D_6$]DMSO, 100 MHz): $\delta = 3,03—3,15$ (m; 3-$CH_2$, 5-$CH_2$, Thiomorpholin-dioxid), 3,65—3,80 (m; 2-$CH_2$, 6-$CH_2$, Thiomorpholin-dioxid), 6,87—6,99 (BB'-System; 3-H, 5-H), 7,16—7,32 ppm (AA'-System, 2-H, 6-H).

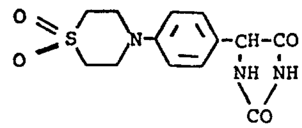

b) 5-(4-Thiomorpholinophenyl)-2,4-imidazolidindion-S,S-dioxid

Analog Beispiel 1 wird aus 87,7 g (0,367 Mol) 4-Thiomorpholino-benzaldehyd-S.S-dioxid, 26,8 g (0,546 Mol) Natriumcyanid und 143 g (1,49 Mol) Ammoniumcarbonat in 1100 ml Wasser und 1100 ml Äthanol das entsprechende Hydantoin hergestellt.

Ausbeute 87 g (77%).

$C_{13}H_{15}N_3O_4S$ (309,3)

| | | | | |
|---|---|---|---|---|
| Ber. | C 50,48 | H 4,89 | N 13,58 | S 10,37 |
| Gef. | C 49,4 | H 5,0 | N 13,9 | S 10,0 |

$^1$H-NMR ([$D_6$]DMSO, 60 MHz): $\delta = 2{,}95$—3,30 (m; 3-$CH_2$, 5-$CH_2$, Thiomorpholin-dioxid), 3,62—3,95 (m; 2-$CH_2$, 6-$CH_2$, Thiomorpholin-dioxid), 7,05 (d, J = 8,4 Hz, 3-H, 5-H), 7,3 ppm (d, J = 8,4 Hz, 2-H, 6-H).

## Beispiel 4

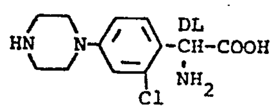

a) DL-$\alpha$-Amino-$\alpha$-[2-Chloro-4-(1-piperazinyl)phenyl]essigsäure

Durch eine Suspension von 20 g Palladiummohr in 350 ml Methanol, in der das Natriumsalz von 19,5 g (0,0362 Mol) DL-$\alpha$-Benzyloxycarbonylamino-$\alpha$-{2-chloro-4-[4-(benzyloxycarbonyl)-1-piperazinyl]phenyl} essigsäure gelöst ist, läßt man 3,5 Stunden Wasserstoff strömen. Dann wird der Katalysator abgetrennt, mit Methanol gewaschen und das Filtrat im Vakuum bis zur Trockene eingeengt.

Ausbeute: 9,6 g (86%).

$C_{12}H_{15}ClN_3NaO_2 \cdot H_2O$ (309,7)

| | | | | |
|---|---|---|---|---|
| Ber. | C 46,54 | H 5,54 | N 13,56 | Cl 11,45 |
| Gef. | C 46,9 | H 5,6 | N 11,7 | Cl 11,5 |

$^1$H-NMR (NaOD, 60 MHz): $\delta = 2{,}67$—3,18 (breites s; 8H, Piperazinyl), 4,32 (s; $\alpha$-CH), 7,03 (d; J = 8,4 Hz, 3-H, 5-H), 7,37 ppm (d; J = 8,4 Hz, 6-H).

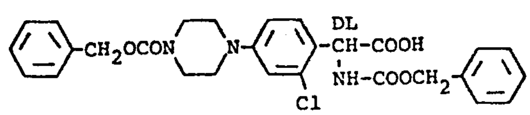

b) DL - $\alpha$ - Benzyloxycarbonylamino - $\alpha$ - {2 - chloro - 4 - [4 - (benzyloxycarbonyl) - 1 - piperzinyl]phenyl}essigsäure

45,0 g (0,167 Mol) DL-$\alpha$-Amino-$\alpha$-[2-chloro-4-(1-piperazinyl)phenyl]essigsäure werden in 200 ml Wasser gelöst und mit 2 N NaOH ein pH-Wert von 8,8—9,0 eingestellt. Die klare Lösung wird auf 0 bis 5°C gekühlt und innerhalb von 50 Minuten werden 61 ml (0,401 Mol) Chlorameisensäurebenzylester unter gleichzeitiger Zugabe von 1 N NaOH zugetropft. Nach kurzer Zeit bildet sich eine schmierige Suspension, die sich durch weitere Zugabe von 200 bis 300 ml 2 N NaOH in eine klare dunkelrot gefärbte Lösung verwandelt. Nunmehr tropft man den Rest an Chlorameisensäurebenzylester bei 5°C ein und läßt über Nacht rühren. Die Reaktionslösung wird dann einmal mit 600 ml Äther extrahiert, die wäßrige Phase, die eine ölige Schicht enthält, abgetrennt, mit konzentrierter Salzsäure bei Eiskühlung auf pH 2 angesäuert und mit Essigester ausgezogen. Nach Waschen mit Wasser, Trocknen mit Natriumsulfat und Einengen der organischen Phase erhält man 66,9 g (74%) Benzyloxycarbonylgeschützte Aminosäure.

$C_{28}H_{28}ClN_3O_6$ (538,0)

| | | | | |
|---|---|---|---|---|
| Ber. | C 62,51 | H 5,25 | N 7,81 | Cl 6,59 |
| Gef. | C 63,6 | H 5,4 | N 7,1 | Cl 6,1 |

$^1$H-NMR ($CDCl_3$, 60 MHz): $\delta$ = 2,85—3,22 (breites s; 2-$CH_2$, Piperazinyl), 3,40—3,71 (breites s; 3-$CH_2$, 5-$CH_2$, Piperazinyl), 4,6 (s; $\alpha$-CH), 5,04 (s; Benzyl-$CH_2$), 5,13 (s; Benzyl-$CH_2$), 6,76—7,50 ppm (breites m; 13H, Phenyl).

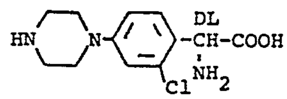

c) DL-$\alpha$-Amino-$\alpha$-[2-chloro-4-(1-piperazinyl)phenyl]essigsäure

67,1 g (0,229 Mol) 5-[2-Chlor-4-(1-piperazinyl)phenyl]-2,4-imidazolidindion werden in 750 ml 48%iger Bromwasserstofflösung 48 Stunden unter Rückfluß erhitzt. Die Lösung wird anschließend im Vakuum bis zur Trockene eingeengt, der Rückstand in 150 ml Wasser gelöst und mit 2 N NaOH ein pH-Wert von 4,5 eingestellt. Die wäßrige Phase wird mit ca. 4 l Äthanol versetzt; dabei fällt die Aminosäure zum Teil in Form einer Paste an.

Ausbeute: 38,1 g (62%).

Aus dem Filtrat werden noch 95,7 g Substanz isoliert.

$^1$H-NMR (NaOD, 60 MHz): $\delta = 2,94$ (breites s; 8H, Piperazinyl), 4,66 (s; $\alpha$-CH), 6,97 (d; J = 8,4 Hz, 3-H, 5-H), 7,33 ppm (d; J = 8,4 Hz, 6-H).

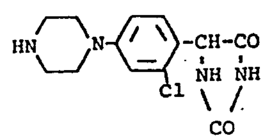

d) 5-[2-Chlor-4-(1-piperazinyl)phenyl]-2,4-imidazolidindion

Das Hydantoin wird analog Beispiel 1 aus 99,6 g (0,384 Mol) 2-Chlor-4-(1-piperazinyl)benzaldehyd hydrochlorid, 28,2 g (0,576 Mol) Natriumcyanid und 312 g (3,26 Mol) Ammoniumcarbonat in 600 ml Wasser und 500 ml Äthanol hergestellt.

Ausbeuten: 1. Fraktion 33,7 g (30%)
2. Fraktion 34,4 g (31%)

$C_{13}H_{14}ClN_4O_2$ (293,7)

| | | | | |
|---|---|---|---|---|
| Ber. | C 53,16 | H 4,81 | N 19,08 | Cl 12,07 |
| Gef. | C 52,5 | H 5,4 | N 16,6 | Cl 14,4 |

$^1$H-NMR ([$D_6$]DMSO; 60 MHz): $\delta = 2,84$—3,50 (breites m; 8H, Piperazinyl), 5,21 (s; 5-H), 6,93—7,41 ppm (m; 3H, Phenyl).

Der 2-Chlor-4-(1-piperazinyl)benzaldehyd wird nach Vorschriften von D. Rosi et al., J. Med. Chem. *10*, 877—880 (1967) hergestellt.

## Beispiel 5

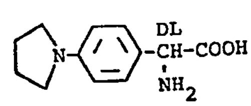

a) DL-$\alpha$-Amino-$\alpha$-(4-pyrrolidino-phenyl)essigsäure

Aus 63,5 g (0,259 Mol) 5-(4-Pyrrolidino-phenyl)-2,4-imidazolidindion gewinnt man analog Beispiel 1 das neue Phenylglycinderivat.

Ausbeute: 22,3 g (37%)

$C_{12}H_{16}N_2O_2$ (220,3)

| | | | |
|---|---|---|---|
| Ber. | C 65,43 | H 7,32 | N 12,71 |
| Gef. | C 64,8 | H 7,2 | N 12,8 |

$^1$H-NMR (NaOD/$D_2O$): $\delta$ = 1,75—2,08 (m; 3-$CH_2$, 4-$CH_2$, Pyrrolidin), 3,0—3,34 (m; 2-$CH_2$, 5-$CH_2$, Pyrrolidin), 4,32 (s; $\alpha$-CH), 6,74 (d; J = 8,3 Hz, 3-H, 5-H), 7,36 ppm (d; J = 8,3 Hz, 2-H, 6-H).

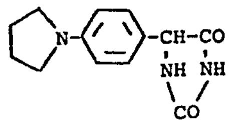

b) 5-(4-Pyrrolidino-phenyl)-2,4-imidazolidindion

Das Hydantoin wird analog Beispiel 1 aus 130,4 g (0,745 Mol) p-Pyrrolidinobenzaldehyd, 55 g (1,12 Mol) Natriumcyanid und 287 g (2,99 Mol) Ammoniumcarbonat hergestellt.

Ausbeute: 127,4 g (74%)

$C_{13}H_{15}N_3O_2$ (245,3)

| | | | |
|---|---|---|---|
| Ber. | C 63,65 | H 6,16 | N 17,13 |
| Gef. | C 62,8 | H 6,2 | N 16,7 |

$^1$H-NMR ([$D_6$]DMSO, 60 MHz): $\delta = 1{,}79$—2,09 (m; 3-$CH_2$, 4-$CH_2$, Pyrrolidino), 3,07—3,36 (m; 2-$CH_2$, 5-$CH_2$, Pyrrolidino), 4,99 (s; 5-H), 6,53 (d; J = 8,3 Hz, 3-H, 5-H), 7,10 ppm (d; J = 8,3 Hz, 2-H, 6-H).

N-Phenylpyrrolidin und p-Pyrrolidinobenzaldehyd werden nach Vorschriften aus Brevet d'Invention No. 616 609 (18.4.1962) der Wellcome Foundation Lmt. hergestellt.

Beispiel 6

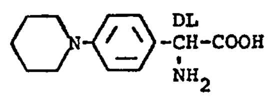

a) DL-$\alpha$-Amino-$\alpha$-(4-piperidino-phenyl)essigsäure

Aus 76,5 g (0,295 Mol) 5-(4-Piperidino-phenyl)-2,4-imidazolidindion gewinnt man analog Beispiel 1 das neue p-Piperidinophenylglycin.

Ausbeute: 51 g (64%)

$C_{13}H_{18}N_2O_2 \cdot 2H_2O$ (270,3)

| | | | |
|---|---|---|---|
| Ber. | C 57,77 | H 8,20 | N 10,37 |
| Gef. | C 56,8 | H 6,8 | H 10,3 |

$^1$H-NMR (DCOOD, 60 MHz): $\delta$ = 1,65—2,34 (m; 3-$CH_2$, 4-$CH_2$, 5-$CH_2$, Piperidino, 3,61—3,92 (m; 2-$CH_2$, 6-$CH_2$, Piperidino), 5,49 (s; $\alpha$-CH), 7,86 ppm (s; 4H, Phenyl).

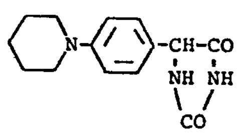

b) 5-(4-Piperidino-phenyl)-2,4-imidazolidindion

Das Hydantoin wird analog Beispiel 1 aus 80 g (0,423 Mol) p-Piperidinobenzaldehyd, 31 g (0,633 Mol) Natriumcyanid und 164 g (1,71 Mol) Ammoniumcarbonat hergestellt.

Ausbeute: 78 g (72%)

$C_{14}H_{17}N_3O_2$ (259,3)

| | | | |
|---|---|---|---|
| Ber. | C 64,85 | H 6,61 | N 16,20 |
| Gef. | C 64,5 | H 6,6 | N 17,2 |

H-NMR ([$D_6$] DMSO, 60 MHz): $\delta$ = 1,60 (breites s; 3-$CH_2$, 4-$CH_2$, 5-$CH_2$, Piperidino), 3,02—3,31 (m; 2-$CH_2$, 6-$CH_2$, Piperdino), 5,05 (s; 5-H), 6,86 (d; J = 8,3 Hz, 3-H, 5-H), 7,34 ppm (d; J = 8,3 Hz, 2-H, 6-H).

Die Darstellung des N-Phenylpiperidins ist von H.D. Nitzschke und H. Budka, Chem. Ber. *88,* 264—268 (1955) beschrieben.

## Beispiel 7

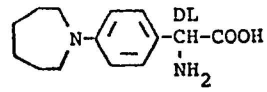

a) DL-$\alpha$-Amino-$\alpha$-(4-hexamethylenimino-phenyl)essigsäure

Aus 55 g (0,202 Mol) 5-(4-Hexamethylenimino-phenyl)-2,4-imidazolidindion gewinnt man analog Beispiel 1 das neue Phenylglycinderivat.

Ausbeute: 31,5 g (59%)

$C_{14}H_{20}N_2O_2 \cdot H_2O$ (266,3)

| | | | |
|---|---|---|---|
| Ber. | C 63,15 | H 8,33 | N 10,52 |
| Gef. | C 62,3 | H 7,5 | N 10,1 |

$^1$H-NMR (NaOD, 60 MHz): $\delta$ = 1,45 (breites s; 8H, Hexamethylenimino), 3,06—3,43 (m; 2-$CH_2$, 7-$CH_2$, Hexamethylenimino), 4,23 (s; $\alpha$-CH), 6,62 (d; J = 8,3 Hz, 3-H, 5-H), 7,26 ppm (d; J = 8,3 Hz, 2-H, 6-H).

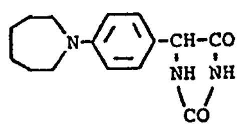

b) 5-(4-Hexamethylenimino-phenyl)-2,4-imidazolidindion

Das Hydantoin wird analog Beispiel 1 aus 55,5 g (0,273 Mol) p-Hexamethylenimino-benzaldehyd, 20,1 g (0,41 Mol) Natriumcyanid und 105,5 g (1,1 Mol) Ammoniumcarbonat hergestellt.

Ausbeute: 65 g (87%)

$C_{15}H_{19}N_3O_2$ (273,3)

| | | | |
|---|---|---|---|
| Ber. | C 65,92 | H 7,01 | N 15,37 |
| Gef. | C 64,5 | H 7,0 | N 15,1 |

$^1$H-NMR ([$D_6$] DMF, 100 MHz): $\delta$ = 1,4—1,96 (m; 8H, Hexamethylenimino), 3,43—3,61 (t, 2-$CH_2$, 7-$CH_2$, Hexamethylenimino), 5,10 (s, 5-H), 6,75 (d; J = 8,5 Hz, 3-H, 5-H), 7,22 ppm (d; J = 8,5 Hz, 2-H, 6-H).

## Beispiel 8

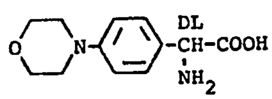

a) DL-$\alpha$-Amino-$\alpha$-(4-morpholinophenyl)essigsäure

Aus 50 g (0,192 Mol) 5-(4-Morpholinophenyl)-2,4-imidazolidindion gewinnt man analog Beispiel 1 das p-Morpholinophenylglycin.

Ausbeute: 29,6 g (60%)

$C_{12}H_{16}N_2O_3 \cdot H_2O$ (254,3)

| | | | |
|---|---|---|---|
| Ber. | C 56,68 | H 7,13 | N 11,02 |
| Gef. | C 57,5 | H 6,8 | N 11,0 |

$^1$H-NMR ($D_2O$/NaOD, 60 MHz): $\delta$ = 2,9—3,2 (m; 3-$CH_2$, 5-$CH_2$, Morpholino), 3,66—3,96 (m; 2-$CH_2$, 6-$CH_2$, Morpholino), 4,30 (s; $\alpha$-CH), 6,98 (d; J = 8,3 Hz, 3-H, 5-H), 7,34 ppm (d; J = 8,3 Hz, 2-H, (6-H).

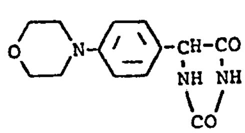

b) 5-(4-Morpholino-phenyl)-2,4-imidazolidindion

Das Hydantoin wird analog Beispiel 1 aus 61,9 g (0,324 Mol) p-Morpholino-benzaldehyd, 23,8 g (0,485 Mol) Natriumcyanid und 126 g (1,31 Mol) Ammoniumcarbonat hergestellt.

Ausbeute: 51,4 g (61%)

| $C_{13}H_{15}N_3O_3$ (261,3) | | | |
|---|---|---|---|
| Ber. | C 59,76 | H 5,79 | N 16,08 |
| Gef. | C 60,4 | H 5,8 | N 16,3 |

$^1$H-NMR ([$D_6$] DMSO, 60 MHz): $\delta$ = 3,0—3,29 (m; 3-$CH_2$, 5-$CH_2$, Morpholino), 3,66—4,93 (m; 2-$CH_2$, 6-$CH_2$, Morpholino), 7,0 (d; J = 8,3 Hz, 3-H, 5-H), 7,29 ppm (d; J = 8,3 Hz, 2-H, 6-H).

Beispiel 9

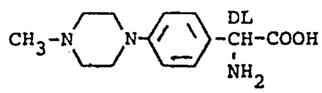

a) DL-$\alpha$-Amino-$\alpha$-[4-(4-methylpiperazin-1-yl)phenyl]essigsaüre oder 1-Phenylglycinyl-4-methylpiperazin

Aus 220 g (0,803 Mol) 5-[4-(4-Methylpiperazin-1-yl)phenyl]-2,4-imidazolidindion gewinnt man analog Beispiel 3 das p-(4-Methylpiperazin-1-yl)phenylglycin.

Aufarbeitung: Die alkalische Lösung wird auf pH 2 bei 0°C angesäuert, danach auf pH 5 zurückgestellt und weitgehend eingeengt (600 ml). Nach Zugabe von ca. 2,5 l Äthanol wird das ausgefällte Material abgesaugt. Ausbeute: 143,5 g (enthält ~75% Natriumchlorid).

Das Filtrat wird nochmals mit 4 l Äthanol versetzt und 3 Tage im Kühlschrank aufbewahrt. Das ausgefallene Produkt wird abgesaugt und getrocknet.

Ausbeute: 96 g (enthält 30% NaCl)

| $C_{13}H_{19}N_3O_2$ (249,3) | | | | |
|---|---|---|---|---|
| Ber. | C 43,7 | H 5,35 | N 11,75 | Cl 18,3 |
| Gef. | C 42,1 | H 6,4 | N 11,6 | Cl 18,3 |

$^1$H-NMR (NaOD, 60 MHz): $\delta$ = 2,28 (s; $CH_3$), 2,46—2,69 (m; 3-$CH_2$, 5-$CH_2$, Piperazinyl), 2,99—3,23 (m; 2-$CH_2$, 6-$CH_2$, Piperazinyl), 4,31 (s; $\alpha$-CH), 6,97 (d; J = 8,4 Hz, 3-H, 5-H). 7,32 ppm (d; J = 8,4 Hz, 2-H, 6-H).

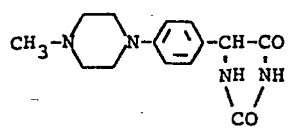

b) 5-[4-(4-Methylpiperazin-1-yl)phenyl]-2,4-imidazolidindion

Das Hydantoin wird analog Beispiel 1 aus 148 g (0,725 Mol) p-(4-Methyl-piperazin-1-yl)-benzaldehyd, 53,4 g (1,09 Mol) Natriumcyanid und 282 g (2,93 Mol) Ammoniumcarbonat hergestellt.

Aufarbeitung: Von der Suspension wird Äthanol im Vakuum abdestilliert und die Restmenge mit konzentrierter Salzsäure auf pH 1,5 angesäuert. Danach wird die klare Lösung mit 4 N NaOH auf pH 7,5—8,0 gestellt und das ausgefallene Produkt abgesaugt, mit Wasser gewaschen und getrocknet.

Ausbeute: 146 g (73%)

| $C_{14}H_{18}N_4O_2$ (274,3) | | | |
|---|---|---|---|
| Ber. | C 61,30 | H 6,61 | N 20,42 |
| Gef. | C 61,4 | H 6,6 | N 20,5 |

$^1$H-NMR ([$D_6$] DMSO, 60 MHz): $\delta$ = 2,23 (s; $CH_3$), 2,34—2,61 (m; 3-$CH_2$, 5-$CH_2$, Piperazinyl), 3,08—3,30 (m; 2-$CH_2$, 6-$CH_2$, Piperazinyl), 5,05 (s; 5-H), 6,97 (d; J = 8,4 Hz, 3-H, 5-H), 7,22 (d; J = 8,4 Hz, 2-H, 6-H).

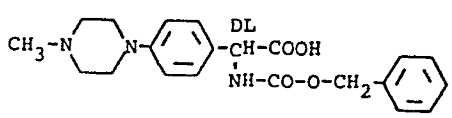

c) DL-α-Benzyloxycarbonylamino-α-[4-(4-methyl-piperazin-1-yl)phenyl]essigsäure

In Anlehnung an eine Vorschrift von Y. Wolman, D. Ladkany und M. Frankel, J.Chem.Soc. *1967*, 689—690, werden 14,6 g (0,0273 Mol) DL-α-Amino-α-[4-(4-methylpiperazin-1-yl)phenyl]essigsäure, die 53,5% Natriumchlorid enthält, in 60 ml Wasser suspendiert, mit 2,3 g (0,0273 Mol) Ammoniumhydrogencarbonat versetzt und 9,0 g (0,033 Mol) p-Nitrophenyl-benzylcarbonat in 60 ml Dioxan zugegeben. Die Reaktionsmischung wird 4 Stunden unter Rückfluß erhitzt. Danach wird die klar gewordene Lösung mit 200 ml Wasser und 200 ml Essigester versetzt, geschüttelt und die Essigesterphase abgetrennt. Aus der wäßrigen Lösung erhält man durch Ansäuern mit 2 N HCl bei 0°C einen Niederschlag, der abgesaugt und getrocknet wird.

Ausbeute: 6,1 g (59%).

$C_{21}H_{25}N_3O_4 \cdot H_2O$ (401,5)

| | | | |
|---|---|---|---|
| Ber. | C 62,82 | H 6,78 | N 10,46 |
| Gef. | C 61,3 | H 6,7 | N 10,2 |

$^1$H-NMR ([$D_6$] DMSO, 60 MHz): δ = 2,31 (s; $CH_3$), 2,42—2,71 (m; 3-$CH_2$, 5-$CH_2$, Piperazinyl), 2,96—3,27 (m; 2-$CH_2$, 6-$CH_2$, Piperazinyl), 5,01 (s; α-CH), 5,17—5,36 (breites s; Benzyl-$CH_2$, -NH-), 6,71—7,31 (AB-System, 4H, Phenyl-H), 7,31 ppm (s; 5H, Phenyl-H).

## Beispiel 10

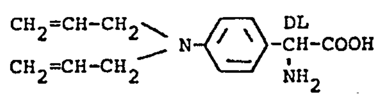

a) DL-α-Amino-α-(4-diallylaminophenyl)essigsäure

Aus 95 g (0,35 Mol) 5-(4-Diallylaminophenyl)-2,4-imidazolidindion gewinnt man analog Beispiel 1 das p-Di-allylamino-phenylglycin. Das Rohmaterial wird zum Schluß in 700 ml siedendem Äthanol verrieben. Nach Abkühlen auf 0°C wird die Aminosäure abgesaugt und mit kaltem Äthanol gewaschen.

Ausbeute: 57,3 g (67%)

$C_{14}H_{18}N_2O_2$ (246,3)

| | | | |
|---|---|---|---|
| Ber. | C 68,28 | H 7,37 | N 11,37 |
| Gef. | C 66,5 | H 8,3 | N 10,5 |

$^1$H-NMR ([$D_6$] DMSO, 60 MHz): δ = 3,86—4,06 (d; 4H, 2-$CH_2$-), 4,22 (s; α-CH), 4,99—5,38 (m; 4H, 2$CH_2$=), 5,60—6,22 (breites m; 2H, 2-CH=), 6,63 (d; J = 8,4 Hz, 3-H, 5-H), 7,21 (d; J = 8,4 Hz, 2-H, 6-H).

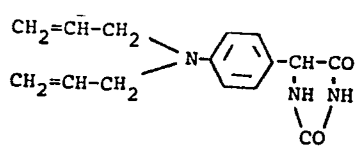

b) 5-[4-(Diallylamino)phenyl]-2,4-imidazolidindion

Das Hydantoin wird analog Beispiel 1 aus 108 g (0,536 Mol) p-Diallylaminobenzaldehyd, 39,7 g (0,81 Mol) Natriumcyanid und 209 g (2,18 Mol) Ammoniumcarbonat hergestellt.

Aufarbeitung: Nach Abdestillieren des Äthanols im Vakuum wird die Restlösung mit halbkonzentrierter Salzsäure auf pH 1,5 angesäuert und 15 Minuten gerührt. Danach wird die Lösung mit 4 N NaOH bei 5 bis 10°C auf pH 7 gestellt. Das ausgefallene Produkt wird abgesaugt, mit Wasser gewaschen und trocken gesaugt. Das Material wird dann dreimal mit Äther verrieben, abgesaugt und im Vakuum getrocknet.

Ausbeute: 95,6 g (66%)

$C_{15}H_{17}N_3O_2$ (271,3)

| | | | |
|---|---|---|---|
| Ber. | C 66,40 | H 6,32 | N 15,49 |
| Gef. | C 65,4 | H 6,4 | N 16,0 |

$^1$H-NMR ([$D_6$] DMSO, 60 MHz): δ = 3,92—4,00 (d; 4H, 2-$CH_2$-), 4,97—5,35 (m; 4H, 2$CH_2$=), 5,70 (s; 5-H), 5,62—6,21 (breites m; 2H, 2-CH=), 6,72 (d; J = 8,4 Hz, 3-H, 5-H), 7,12 ppm (d; J = 8,4 Hz, 2-H, 6-H).

Der p-Diallylamino-benzaldehyd wird aus dem p-Diallylanilin über die Vilsmeier-Reaktion dargestellt. Die Synthese des Diallylanilins ist bei Ch.L. McCormick und G.B. Butler, J.Org.Chem. *41,* 2803—2808 (1976) beschrieben.

Beispiel 11

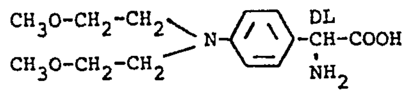

a) DL-$\alpha$-Amino-$\alpha$-[4-bis($\beta$-methoxyethyl)aminophenyl] essigsäure

Aus 70 g (0,0228 Mol) 5-[4-Bis($\beta$-methoxy-ethyl)aminophenyl]-2,4-imidazolidindion gewinnt man analog Beispiel 3 das neue Phenylglycinderivat.

Aufarbeitung: Man kühlt auf 0°C ab, säuert mit konzentrierter Salzsäure auf pH 1 an und rührt 15 Minuten. Danach wird der pH-Wert der Lösung auf pH 4,6 gebracht, wobei das ausgefallene Produkt (5,2 g) abgesaugt und verworfen wird. Das Filtrat wird im Vakuum eingeengt und der Rückstand mit 2 l Methanol 2 bis 3 Stunden bei Raumtemperatur verrührt. Danach wird vom unlöslichen Material (Natriumchlorid) abgesaugt und das methanolische Filtrat im Vakuum bis zur Trockene eingeengt.

Ausbeute: 60,2 g (94%; enthält 14,8% Natriumchlorid).

$C_{14}H_{22}N_2O_4$ (282,3)

| | | | | |
|---|---|---|---|---|
| Ber. | C 50,8 | H 6,7 | N 8,47 | |
| Gef. | C 49,6 | H 6,4 | N 9,0 | Cl 9,0 |

$^1$H-NMR (NaOD, 60 MHz); $\delta$ = 3,29 (s; 6H, 2$CH_3O$), 3,48 (s; 8H, 4 $CH_2$), 4,26 (s; $\alpha$-CH), 6,72 (d; J = 8,4 Hz, 3-H, 5-H), 7,25 ppm (d; J = 8,4 Hz, 2-H, 6-H).

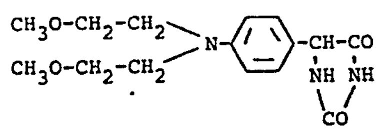

b) 5-[4-Bis($\beta$-methoxyethyl)aminophenyl]-2,4-imidazolidindion

Das Hydantoin wird analog Beispiel 1 aus 108 g (0,455 Mol) N,N-Bis($\beta$-methoxyethyl)aminobenzaldehyd, 33,7 g (0,688 Mol) Natriumcyanid und 177 g (1,84 Mol) Ammoniumcarbonat hergestellt.

Aufarbeitung: Nach 20 Stunden Rühren bei 60°C wird Äthanol im Vakuum abdestilliert. Die Restlösung wird bei Eiskühlung mit halbkonzentrierter Salzsäure auf pH 1,5 angesäuert und nach 10 Minuten Rühren mit 4 N NaOH auf pH 7,2 gestellt. Die wäßrige Suspension wird gründlich mit Chloroform extrahiert, die Chloroform-Phasen gewaschen und getrocknet. Nach Einengen der organischen Phase erhält man eine zähe, viskose Masse.

Ausbeute: 72 g (51%)

$C_{15}N_{21}N_3O_4$

| | | | |
|---|---|---|---|
| Ber. | C 58,61 | H 6,88 | N 13,67 |
| Gef. | C 58,8 | H 6,9 | N 13,5 |

$^1$H-NMR ($CDCl_3$, 60 MHz): $\delta$ = 3,34 (s; 6H, 2 $CH_3O$), 3,55 (s; 8H, 4 $CH_2$), 4,93 (s; 5-H), 6,57—7,29 ppm (AA'BB'-System; 4H, Phenyl).

Der N,N-Bis($\beta$-methoxyethyl)aminobenzaldehyd wird aus dem Bis-($\beta$-methoxyethyl)anilin, über die Vilsmeier-Reaktion dargestellt. Das Bis-($\beta$-methoxyethyl)-anilin wird nach W.R. Boon, J.Chem.Soc. *1147,* 307—318 erhalten.

Beispiel 12

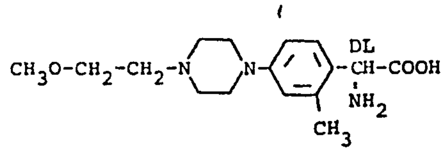

a) DL-$\alpha$-Amino-$\alpha$-[4-(4-$\beta$-methoxyethyl-piperazin-1-yl-2-methylphenyl]essigsäure

Aus 42 g (0,127 Mol) 5-[4-(4-$\beta$-Methoxyethyl-piperazin-1-yl)-2-methylphenyl]-2,4-imidazolidindion gewinnt man analog Beispiel 11 das Phenylglycinderivat.

Ausbeute: 40 g (103%, enthält 30,6% Natriumchlorid).

$C_{16}H_{25}N_3O_3$ (307,4)

| | | | |
|---|---|---|---|
| Ber. | C 43,3 | H 5,69 | N 9,5 |
| Gef. | C 43,0 | H 6,4 | N 11,0 |

$^1$H-NMR [[$D_6$] DMSO/$D_2O$, 60 MHz): $\delta$ = 2,4 (s; 2-$CH_3$), 3,04—3,4 (m; 8H, Piperazinyl); 2 H, $\alpha$-$CH_2$-), 3,32 (s; $CH_3O$-), 3,59—3,79 (m; 2H, $\beta$-$CH_2$-), 4,6 (s; $\alpha$-CH), 6,66—7,4 ppm (m; 3H, Phenyl).

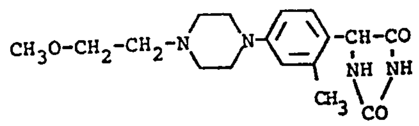

b) 5-[4-(4-$\beta$-Methoxyethyl-piperazin-1-yl)-2-methylphenyl]-2,4-imidazolidindion

Das Hydantoin wird analog der Beispiele 1 und 11 aus 58,7 g (0,224 Mol) 4-[4-($\beta$-Methoxyethyl)piperazin-1-yl]-2-methylbenzaldehyd, 16,6 g (0,339 Mol) Natriumcyanid und 88 g (0,916 Mol) Ammoniumcarbonat hergestellt.

Ausbeute: 42 g (57%)

$C_{17}H_{24}N_4O_3$ (332,4)

| | | | |
|---|---|---|---|
| Ber. | C 61,43 | H 7,78 | N 16,86 |
| Gef. | C 60,8 | H 7,2 | N 16.4 |

$^1$H-NMR ($CD_3OD$, 60 MHz): $\delta$ = 2,35 (s; 2-$CH_3$), 2,63 (d, 3-$CH_2$, 5-CH, Piperazinyl; 2H, $\alpha$-$CH_2$-), 3,03—3,25 (m; 2-$CH_2$, 6-$CH_2$, Piperazinyl), 3,31 (s; $CH_3O$-), 3,45—3,63 (t, 2H, $\beta$-$CH_2$-), 5,25 (s; $\alpha$-CH), 6,58—7,15 ppm (m; 3H, Phenyl).

Der 4-[4-($\beta$-Methoxyethyl)piperazin-1-yl]-2-methylbenzyaldehyd wird aus dem N-(2-Methylphenyl)-N'-(2-methoxyethyl)-piperazin über die Vilsmeier-Reaktion dargestellt.

Die Synthese des N-(2-Methylphenyl)-N'-(2-methoxyethyl)-piperazins ist in U.S. Patent 2 891 063, Abbott Laboratories (16.6.1959) beschrieben.

Beispiel 13

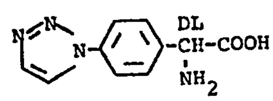

a) DL-$\alpha$-Amino-$\alpha$-[4-(1,2,3-triazol-1-yl)phenyl]essigsäure

Aus 39,5 g (0,172 Mol) 5-[4-(1,2,3-Triazol-1-yl)phenyl]-2,4-imidazolidindion gewinnt man analog Beispiel 1 das neue Phenylglycinderivat.

Ausbeute: 24,5 g (65%; enthält 11,8% Natriumchlorid).

$C_{10}H_{10}N_4O_2 \cdot 2H_2O$ (254,3)

| | | | | |
|---|---|---|---|---|
| Ber. | C 41,7 | H 4,82 | N 19,4 | |
| Gef. | C 41,4 | H 3,9 | N 19,4 | Cl 7,2 |

$^1$H-NMR (NaOD/$CD_3OD$; 60 MHz); $\delta$ = 4,57 (s; $\alpha$-CH), 7,75 (s; 5H, Phenyl-H, Triazolyl-H), 8,0 ppm (s; 1H, Triazolyl-H).

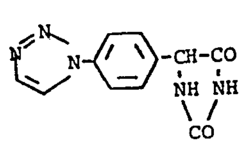

b) 5-[4-(1,2,3-Triazol-1-yl)phenyl]-2,4-imidazolidindion

Das Hydantoin wird analog Beispiel 1 aus 57 g (0,33 Mol) p-(1,2,3-Triazol-1-yl)benzaldehyd 28,5

g (0,583 Mol) Natriumcyanid und 144 g (1,5 Mol) Ammoniumcarbonat in 300 ml Methanol und 300 ml Wasser hergestellt.

Ausbeute: 65,7 g (75%)

| $C_{11}H_9N_5O_2 \cdot 2H_2O$ (265,3) | | | |
|---|---|---|---|
| Ber. | C 49,80 | H 4,94 | N 26,40 |
| Gef. | C 50,7 | H 4,3 | N 25,2 |

$^1$H-NMR ([$D_6$] DMSO, 60 MHz): $\delta$ = 5,4 (s; $\alpha$-CH), 7.57—8,9 ppm (breites m; 4H, Phenyl-H; 2H, Triazolyl-H).

Beispiel 14

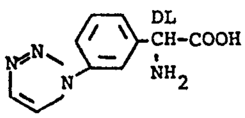

a) DL-$\alpha$-Amino-$\alpha$-[3-(1,2,3-triazol-1-yl)phenyl]essigsäure

Aus 60.4 g (0,248 Mol) 5-[3-(1,2,3-Triazol-1-yl)phenyl]-2,4-imidazolidindion gewinnt man analog Beispiel 11 das m-(1,2,3-Triazol-1-yl)phenylglycin.

Ausbeute: 32,5 g (60%)

| $C_{10}H_{10}N_4O_2$ (218,2) | | | |
|---|---|---|---|
| Ber. | C 55,05 | H 4,62 | N 25,68 |
| Gef. | C 54,5 | H 4,3 | N 24,8 |

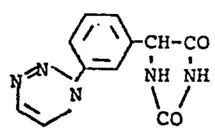

b) 5-[3-(1,2,3-Triazol-1-yl)phenyl]-2,4-imidazolidindion

Das Hydantoin wird analog Beispiel 1 aus 61 g (0,352 Mol) m-(1,2,3-Triazol-1-yl)-benzaldehyd, 26,2 g (0,535 Mol) Natriumcyanid und 137,8 g (1,435 Mol) Ammoniumcarbonat in 1000 ml Wasser und 1000 ml Äthanol hergestellt.

Aufarbeitung: Nach 24 Stunden bei 60°C wird die Reaktionslösung unter Eiskühlung mit 2 N Salzsäure auf pH 2 angesäuert, danach der pH-Wert mit 4 N NaOH auf 4 zurückgestellt und erst dann Äthanol im Vakumm zum großen Teil abdestilliert. Beim Abkühlen auf 0°C fällt festes Material an, das abgesaugt und mit Wasser gewaschen wird.

Ausbeute: 33,9 g (40%)

Das Filtrat wird weiter im Vakuum eingeengt, wobei schmieriges Produkt anfällt, das sich nach kurzer Zeit verfestigt.

Ausbeute: 26,3 g (31%)

| $C_{11}H_9N_5O_2$ (243,2) | | | |
|---|---|---|---|
| Ber. | C 54,33 | H 3,73 | N 28,80 |
| Gef. | C 54,3 | H 3,7 | N 28,4 |

$^1$H-NMR ([$D_6$] DMSO, 60 MHz): $\delta$ = 5,36 (s; $\alpha$-CH), 7,46—8,00 (breites m; 4H, Phenyl; 1H, Triazolyl), 8,77 ppm (s; 1Triazolyl-H).

Beispiel 15

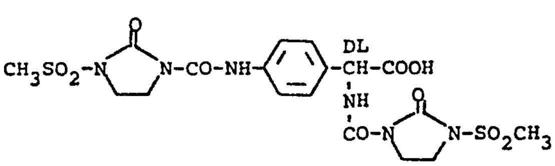

a) DL - $\alpha$ - [(3-Methylsulfonyl-imidazolidin - 2 - on - 1 - yl)carbonylamino] - $\alpha$ - 4 - [(3 - Methylsulfonyl - imidazolidin - 2 - on - 1 - yl)carbonylamino] phenylessigsäure

10 g (0,06 Mol) DL-α-Amino-α-(p-aminophenyl)essigsäure werden in 100 ml Wasser und 100 ml THF unter Zusatz von 2 N NaOH bei pH 7,5 bis 7,8 in Lösung gebracht. Man fügt unter Eiskühlung portionsweise 1-Chlorcarbonyl-3-methylsulfonyl-imidazolidinon-(2) zu und hält durch gleichzeitige Zugabe von 2 N NaOH den pH-Wert von 7,5 aufrecht. Man rührt anschließend noch 2,5 Stunden ohne Kühlung bei pH 7,5 nach.

Aufarbeitung: THF wird abdestilliert, die Restlösung mit 150 ml Wasser versetzt und einmal mit Essigester extrahiert. Die wäßrige Phase wird abgetrennt, mit 2 N ClH auf pH 2 angesäuert und mit Essigester ausgezogen. Nach Waschen mit Wasser, Trocknen mit Natriumsulfat und Einengen der organischen Phase erhält man 10,9 g Rohprodukt, das aus THF/Petroläther umkristallisiert wird.

Ausbeute: 9,0 g (27%)

$C_{18}H_{22}N_6O_{10}S_2$ (546,5)

| | | | | |
|---|---|---|---|---|
| Ber. | C 39,56 | H 4,05 | N 15,37 | S 11,75 |
| Gef. | C 39,4 | H 4,2 | N 14,4 | S 11,2 |

$^1$H-NMR ([$D_6$] DMSO, 60 MHz): δ = 3,39 (s; 6H, 2 $CH_3$), 3,73—4,96 (m; 8 H, Imidazolidinon), 5,08 (d; α-CH), 7,36 (d; J = 8,3 Hz, 3-H,5-H), 7,58 ppm (d; J = 8,3 Hz, 2-H, 6-H).

Die Synthese des 1-Chlorcarbonyl-3-methylsulfonylimidazolidinons-(2) ist in DOS 2 152 968, BAYER AG, Leverkusen (23.10.1971) beschrieben.

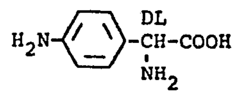

b) DL-α-Amino-α-(4-aminophenyl)essigsäure

Aus 70 g (0,366 Mol) 5-(4-Aminophenyl)-2,4-imidazolidindion gewinnt man analog Beispiel 1 das p-Aminophenylglycin.

Aufarbeitung: Nach 29 Stunden Rühren bei 100°C mit 10%iger Natronlauge wird die Lösung auf 0°C abgekühlt und mit konzentrierter Salzsäure auf pH 2,5 angesäuert. Anschließend wird die wäßrige Lösung auf pH 5,0 gestellt und im Vakuum auf ein kleines Volumen eingeengt, wobei nach Eiskühlung die Aminosäure ausfällt. Der Niederschlag wird abgesaugt, mit wenig Wasser gewaschen und getrocknet.

Ausbeute: 57,3 g (94%)

$C_8H_{10}N_2O_3$ (166,2)

| | | | |
|---|---|---|---|
| Ber. | C 57,82 | H 6,06 | N 16,86 |
| Gef. | C 57,4 | H 6,2 | N 16,6 |

$^1$H-NMR ([$D_6$] DMSO/NaOD, 60 MHz): δ = 4,1 (s; α-CH), 6,63 (d; J = 8,3 Hz, 3-H, 5-H), 7,15 ppm (d; J = 8,3 Hz, 2-H, 6-H).

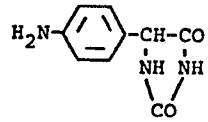

c) 5-(4-Aminophenyl)-2,4-imidazolidindion

Das Hydantoin wird analog Beispiel 9 aus 90 g (0,495 Mol Trockensubstanz angenommen) wasserfeuchtem p-Aminobenzaldehyd, 37,0 g (0,75 Mol) Natriumcyanid und 193,9 g (2,02 Mol) Ammoniumcarbonat in 100 ml Wasser und 1000 ml Äthanol hergestellt.

Ausbeute: 69,4 (73,3%)

$C_9H_9N_3O_2$ (191,2)

| | | | |
|---|---|---|---|
| Ber. | C 56,54 | H 4,74 | N 21,99 |
| Gef. | C 56,8 | H 4,7 | N 21,8 |

$^1$H-NMR ([$D_6$] DMSO, 60 MHz): δ = 4,95 (s; 5-H), 6,65 (d; J = 8,3 Hz, 3-H, 5-H), 7,03 (d; J = 8,3 Hz, 2-H, 6-H).

Beispiel 16

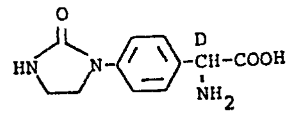

a) D-$\alpha$-Amino-$\alpha$-[4-(imidazolidin-2-on-1-yl)phenyl]essigsäure

18 g (0,0537 Mol) D-$\alpha$-tert.-Butyloxycarbonylamino-$\alpha$-[4-(imidazolidin-2-on-1-yl)phenyl]essigsäure werden in 120 ml Trifluoressigsäure und 40 ml Anisol 30 Minuten bei Raumtemperatur gerührt. Dann wird die Trifluoressigsäure im Vakuum abdestilliert und der ölige Rückstand mit 300 ml Äther versetzt. Das ausgefallene Kristallisat wird abgesaugt, mit Äther gewaschen und im Exsikkator über KOH getrocknet.

Ausbeute: 23,2 g (93%)

$C_{11}H_{13}N_3O_3 \cdot CF_3$ COOH (349,3)

| | | | |
|---|---|---|---|
| Ber. | C 44,70 | H 4,33 | N 12,03 |
| Gef. | C 45,3 | H 4,3 | N 11,3 |

$^1$H-NMR ($D_2O$, 60 MHz): $\delta$ = 3,36—3,94 (m; 4H, Imidazolidinon), 7,26—7,85 (m; 4H, Phenyl).

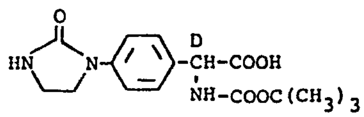

b) D-$\alpha$-tert.-Butyloxycarbonylamino-$\alpha$-[4-(imidazolidin-2-on-1-yl)phenyl]essigsäure

49 g (0,127 Mol) D-$\alpha$-tert.-Butyloxycarbonylamino-$\alpha$-{4-[3-($\beta$-chloräthyl)-ureido]phenyl} essigsäure werden portionsweise innerhalb von 60 Minuten in eine äthanolische Kaliumhydroxidlösung [28,4 g] (0,508 Mol) KOH in 260 ml 70%igem Äthanol] eingetragen und danach 60 Minuten unter Rückfluß erhitzt. Anschließend wird der Ansatz auf 2 1 Eiswasser geschüttet und mit 2 N HCl auf pH 2,8 angesäuert. Das ausgefallene, schmierige Material wird mit Essigester extrahiert, die Essigesterextrakte einmal mit Wasser gewaschen, über Natriumsulfat getrocknet und das organische Lösungsmittel im Vakuum abdestilliert.

Ausbeute: 31,7 g (73%)

$C_{16}H_{21}N_3O_5$ (335,4)

| | | | |
|---|---|---|---|
| Ber. | C 57,30 | H 6,31 | N 12,53 |
| Gef. | C 57,1 | H 4,9 | N 11,3 |

$^1$H-NMR ($CD_3OD$, 60 MHz): $\delta$ = 1,44 (s; 9H, tert.-Butyl), 3,26—3,64 (m; 4-$CH_2$, Imidazolidinon), 3,64—4,06 (m; 5-$CH_2$, Imidazolidinon), 5,16 (s; $\alpha$-CH), 7,11—7,59 ppm (breites m, 4H, Phenyl).

D-$\alpha$-tert.-Butyloxycarbonylamino-$\alpha$-{4-[3-($\beta$-chloräthyl)ureido]phenyl}essigsäure wird durch Kondensation von Chloräthylisocyanat an D-$\alpha$-tert.-Butyloxycarbonylamino-$\alpha$-(p-aminophenyl)essigsäure hergestellt.

Beispiel 17 (Racemattrennung)

Eine Lösung von 25,2 g DL-$\alpha$-Amino-$\alpha$-(4-thiomorpholinophenyl)-essigsäure (Beispiel 1) in 210 ml Ameisensäure werden unter Kühlung mit 70 ml Acetanhydrid versetzt und nach ca. 1 Stunde durch Zugabe von 80 g Eis hydrolysiert. Nach Abziehen des Lösungsmittels wird der harzige Rückstand in Wasser aufgenommen, mit Aktivkohle behandelt und nach Filtration stufenweise eingeengt. Die sich bei der Einengung bildenden schmierigen Niederschläge werden jeweils durch Filtration entfernt, bis sich keine Niederschläge mehr bilden. Das Filtrat wird zur Trockne eingedampft und aus Methanol/Äther umgefällt. Man erhält 15,7 g (56% der Theorie) des Formylderivatives der Ausgangsverbindung. Fp. 148°C.

Die so erhaltene Formylverbindung wird mit Dehydroabietylamin als Hilfsbase in an sich bekannter Weise in Äthanol umgesetzt. Aufgrund ihrer unterschiedlichen Löslichkeit werden dann die beiden Diastereomeren des Salzpaares fraktioniert kristallisiert. Man erhält 8,95 g (41,6% der Theorie) eines in Methanol rechtsdrehenden Salzes und 8,25 g (38,3% der Theorie) eines in Methanol linksdrehenden Salzes.

a) Das rechtsdrehende Salz (6,9 g) wird in 300 ml Wasser mit 60 ml konzentriertem Ammoniak versetzt. Das hierbei freiwerdende Dehydroabietylamin wird mit Methylenchlorid extrahiert, die wässrige Lösung zur Trockne eingedampft und das verbleibende Ammoniumsalz mit 35 ml 2 normaler Salzsäure versetzt und 5 Min. auf 50°C erwärmt. Nach Abkühlung auf 5°C werden 140 ml halbkonzentriertes Ammoniak zugegeben und anschließend im Vakuum eingedampft, wobei die L-$\alpha$-Amino-$\alpha$-(4-thiomorpholinophenyl)essigsäure ausfällt, welche mit Wasser und Äther gewaschen wird. Man erhält 1,7 g der optisch aktiven Verbindung des Beispiels 1 vom Schmelzpunkt 229°C.

Drehwert: $[\alpha]_D^{20} = +\ 101.5°$ (c = 1, nHCl).

b) 8,2 g des linksdrehenden Dehydroabietylaminsalzes werden in 450 ml Wasser mit 90 ml konzentriertem Ammoniak versetzt. Das abgespaltene Dehydroabietylamin wird mit Methylenchlorid extrahiert und die wässrige Phase zur Trockne eingedampft. Man erhält 4,35 g des Ammoniumsalzes, welches in einem Gemisch aus 25 ml Wasser und 10 ml Äthanol gelöst wird und mit 2,0 g D-+- Phenäthylamin versetzt wird. Nach Abdampfen des Lösungsmittels und Umkristallisieren aus Isopropanol erhält man 4,0 g des D-+- Phenäthylaminsalzes vom Schmelzpunkt 177—178°C (Drehwert: $[\alpha]_D^{20} = -135{,}6°C$ (c = 1, Methanol)).

Dieses Salz wird anschließend in 160 ml Wasser gelöst und mit 63 ml konzentriertem Ammoniak versetzt. Anschließend wird das Phenäthylamin mit Methylenchlorid extrahiert, wie wässrige Phase zur Trockne eingedampft und der Rückstand mit 56 ml 2 n Salzsäure aufgenommen und 7 Min. auf 50° erwärmt. Nach Abkühlen auf 5'C wird mit 200 ml halbkonzentriertem Ammoniak versetzt. Beim Abziehen des Ammoniaks im Vakuum kristallisiert die linksdrehende Aminosäure aus. Man erhält 1,5 g D-$\alpha$-Amino-$\alpha$-(4-thiomorpholino-phenyl)essigsäure vom Schmelzpunkt 227°C. Drehwert: $[\alpha]_D^{20} = -100{,}0°$ (c = 1, n HCl).

## Patentansprüche

1. $\alpha$-Aminophenylessigsäurederivate der allgemeinen Formel

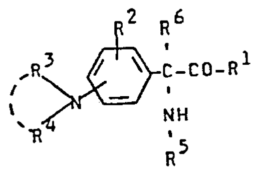

(I)

in welcher

$R^1$ für Hydroxy oder Alkoxy mit 1 bis 4 Kohlenstoffatomen steht,

$R^2$ für Wasserstoff, Alkyl mit 1 bis 4 Kohlenstoffatomen, Trifluormethyl oder Halogen steht,

$R^3$ und $R^4$ gleich oder verschieden und jeweils für Wasserstoff, Allyl oder Alkyl mit bis zu 4 Kohlenstoffatomen stehen, wobei die Alkylreste gegebenenfalls substituiert sind durch Amino, Cyano oder Alkoxy mit 1 bis 2 Kohlenstoffatomen oder

$R^3$ und $R^4$ gemeinsam mit dem Stickstoffatom einen 5-bis 7-gliedrigen aliphatischen Ring bilden, der gegebenenfalls gesättigt oder ungesättigt sein kann, und der ein Sauerstoffatom, ein Schwefelatom, eine SO-Gruppierung, eine $SO_2$-Gruppierung oder eine N-R'''-Gruppierung, wobei R''' für Wasserstoff, Alkyl mit 1 bis 2 Kohlenstoffatomen oder Methoxyäthyl steht, enthalten kann oder gemeinsam mit dem Stickstoffatom einen Triazolylrest bilden,

$R^5$ für Wasserstoff der Alkyl mit 1 bis 4 Kohlenstoffatomen steht und

$R^6$ für Wasserstoff oder Alkyl mit 1 bis 2 Kohlenstoffatomen steht,

sowie ihre pharmakologisch unbedenklichen Salze zur Verwendung bei der Behandlung von Entzündungen.

2. $\alpha$-Amino-$\alpha$-(4-thiomorpholino-phenyl)essigsäure in Form ihrer Racematform oder in Form ihrer optischen Antipoden sowie ihre pharmazeutische unbedenklichen Salze.

3. DL - $\alpha$ - [(3 - Methylsulfonyl - imidazolidin - 2 - on - 1 - yl)carbonyl - amino] - $\alpha$ .- [4 - (3 - methylsulfonyl - imidazolidin - 2 - on - 1 - yl)carbonylamino - phenyl]essigsäure.

4. D-$\alpha$-Amino-$\alpha$-[4-(imidazolidin-2-on1-yl)phenyl]essigsäure.

5. Arzneimittel enthaltend mindestens ein $\alpha$-Aminophenylessigsäurederivt der allgemeinen Formel I gemäß Anspruch 1.

6. Verfahren zur Herstellung von Arzneimitteln, dadurch gekennzeichnet, daß man $\alpha$-Aminophenylessigsäurederivate der allgemeinen Formel I gemäß Anspruch 1 gegebenenfalls unter Verwendung von üblichen Hilfs- und Trägerstoffen in eine geeignete Applikationsform überführt.

7. Verfahren zur Herstellung von $\alpha$-Amino-$\alpha$-(4-thiomorpholinophenyl)essigsäure, dadurch gekennzeichnet, daß man 4-(Thiomorpholino)benzaldehyd in Gegenwart von Natriumcyanid und Ammoniumcarbonat zu dem Hydantoinderivat $\alpha$-Amino-$\alpha$-[4-(Tetrahydro-1,4-thiacenyl)phenyl]essigsäure umsetzt und dieses dann zu der entsprechenden Aminosäure sauer oder alkalisch hydrolysiert.

8. Verfahren gemäß Anspruch 7, dadurch gekennzeichnet, daß man die Reaktion bei Temperaturen zwischen 10 und 120°C durchführt.

9. Verfahren gemäß Anspruch 7, dadurch gekennzeichnet, daß man Natriumcyanid und Ammoniumcarbonat in einem 2- bis 5-fachen Überschuß einsetzt.

**Revendications**

1. Dérivés d'acide $\alpha$-aminophénylacétique de formule générale:

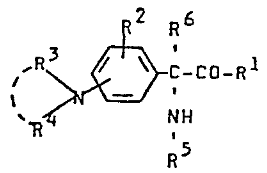

(I)

dans laquelle:

$R^1$ est le groupe hydroxy ou un groupe alkoxy ayant 1 à 4 atomes de carbone,

$R^2$ est l'hydrogène, un groupe alkyle ayant 1 à 4 atomes de carbone, le groupe trifluorométhyle ou un halogène,

$R^3$ et $R^4$ sont égaux ou différents et représentent chacun de l'hydrogène, le reste allyle ou un reste alkyle ayant jusqu'à 4 atomes de carbone, les restes alkyle étant éventuellement substitués par un radical amino, cyano ou alkoxy ayant 1 ou 2 atomes de carbone,

$R^3$ et $R^4$ forment conjointement avec l'atome d'azote un noyau aliphatique pentagonal à heptagonal qui peut éventuellement être saturé ou non saturé, et qui peut comporter un atome d'oxygène, un atome de soufre, un groupe SO, un groupe $SO_2$ ou un groupe, N-R''', où R''' représente l'hydrogène, un reste alkyle ayant 1 ou 2 atomes de carbone ou le reste méthoxyéthyle, ou forment en commun avec l'atome d'azote un reste triacolyle,

$R^5$ est l'hydrogène ou un reste alkyle ayant 1 à 4 atomes de carbone et

$R^6$ est l'hydrogène ou un reste alkyle ayant 1 ou 2 atomes de carbone,

ainsi que leurs sels pharmacologiquement acceptables destinés à être utilisés dans le traitement d'inflammations.

2. L'acide $\alpha$-amino-$\alpha$-(4-thiomorpholinophényl)-acétique sous la forme de son racémate ou sous la forme de ses antipodes optiques ainsi que leurs sels pharmaceutiquement acceptables.

3. L'acide DL - $\alpha$ - [(3 - méthylsulfonylimidazolidine - 2 - one - 1 - yl)carbonylamino] - $\alpha$ - [4-(3-méthylsulfonyl) - imidazolidine - 2 - one - 1 - yl)carbonylaminophényle] acétqiue.

4. L'acide D-$\alpha$-amino-$\alpha$-[4-(imidazolidine-2-one-1-yl)phényl] acétique.

5. Médicament contenant au moins un dérivé d'acide $\alpha$-aminophénylacétique de formule générale I suivant la revendication 1.

6. Procédé de production de médicaments, caractérisé en ce qu'on transforme des dérivés d'acide $\alpha$-aminophénylacétique de formule générale I suivant la revendication 1, éventuellement en utilisant des adjuvants et supports classiques, en une forme d'application appropriée.

7. Procédé de production d'acide $\alpha$-amino-$\alpha$-(4-thiomorpholinophényl)acétique, caractérisé en ce qu'on transforme le 4-(thiomorpholino)benzahdéhyde en présence de cyanure de sodium et de carbonate d'ammonium en le dérivé d'hydantoïne consistant en l'acide $\alpha$-amino-$\alpha$-[4-(tétrahydro-1,4-thiacényl)phényl]acétique, puis on hydrolyse celui-ci en milieu acide ou alcalin en l'acide aminé correspondant.

8. Procédé suivant la revendication 7, caractérisé en ce qu'on conduit la réaction à des températures comprises entre 10 et 120°C.

9. Procédé suivant la revendication 7, caractérisé en ce qu'on utilise le cyanure de sodium et le carbonate d'ammonium en un excès d'un facteur 2 à 5.

**Claims**

1. $\alpha$-Aminophenylacetic acid derivatives of the general formula

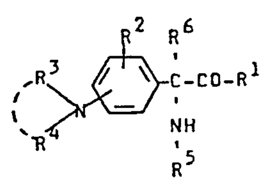

(I)

in which

$R^1$ represents hydroxy or alkoxy with 1 to 4 carbon atoms,

$R^2$ represents hydrogen, alkyl with 1 to 4 carbon atoms, trifluoromethyl or halogen,

$R^3$ and $R^4$ are identical or different and in each case represent hydrogen, allyl or alkyl with up to 4 carbon atoms, the alkyl radicals being optionally substituted by amino, cyano or alkoxy with 1 to 2 carbon atoms or

$R^3$ and $R^4$ together with the nitrogen atom form a 5-membered to 7-membered aliphatic ring, which can optionally be saturated or unsaturated and which can contain one oxygen atom, one sulphur atom, one SO grouping, one $SO_2$ grouping or one N-R''' grouping, wherein R''' represents hydrogen, alkyl with 1 to 2 carbon atoms or methoxyethyl, or together with the nitrogen atom form a triacolyl radical.

$R^5$ represents hydrogen or alkyl with 1 to 4 carbon atoms and

$R^6$ represents hydrogen or alkyl with 1 to 2 carbon atoms,

and pharmacologically acceptable salts thereof, for use in the treatment of inflammatory diseases.

2. $\alpha$-Amino-$\alpha$-(4-thiomorpholinophenyl)-acetic acid in the form of its racemate form or in the form of its optical antipodes and pharmaceutically acceptable salts thereof.

3. DL - $\alpha$ - [(3 - Methylsulphonyl-imidazolidin - 2 - on - 1 - yl) - carbonylamino] - $\alpha$ - [4 - (3 - methylsulphonyl) - imidazolidin - 2 - on - 1 - yl) - carbonylamino - phenyl] - acetic acid.

4. D-$\alpha$-Amino-$\alpha$-[4-(imidazolidin-2-on-yl)-phenyl]-acetic acid.

5. Medicaments containing at least one $\alpha$-aminophenylacetic acid derivative of the general formula I according to Claim 1.

6. Process for the preparation of medicaments, characterised in that $\alpha$-aminophenylacetic acid derivatives of the general formula I according to Claim 1 are converted into a suitable form of administration using, if appropriate, customary auxiliaries and excipients.

7. Process for the preparation of $\alpha$-amino-$\alpha$-(4-thiomorpholinophenyl)-acetic acid, characterised in that 4-(thiomorpholino)-benzaldehyde is reacted in the presence of sodium cyanide and ammonium carbonate to give the hydantoin derivative $\alpha$-amino-$\alpha$-[4-(tetrahydro-1,4-thiacenyl)-phenyl]-acetic acid and this is then hydrolysed by acid or alkaline hydrolysis to the corresponding aminoacid.

8. Process according to Claim 7, characterised in that the reaction is carried out at temperatures between 10 and 120°C.

9. Process according to Claim 7, characterised in that sodium cyanide and ammonium carbonate are employed in a 2-fold to 5-fold excess.